# EUROPEAN PATENT APPLICATION

(11) **EP 4 704 108 A1**
(43) Date of publication of application: **04.03.2026**
(21) Application number: 24197913.7
(22) Date of filing: 02.09.2024
(51) Int. Cl.: G16H 40/40

(54) **METHOD AND SYSTEM FOR SUPPORTING A COMMUNICATION ENGAGEMENT WITH A PLURALITY OF CLASSES OF IMPLANTABLE MEDICAL DEVICES**

(71) Applicant: BIOTRONIK SE & Co. KG, 12359 Berlin (DE)
(72) Inventor: Taff, Brian M., Portland, 97217 (US); Bergstrom, Dean, West Linn, 97068 (US); Swenson, Kurt, Corvallis, 97330 (US); Merlin, Julian, 10779 Berlin (DE)
(74) Representative: Biotronik Corporate Services SE

(57) **Abstract**

The invention relates to a method and system for supporting a communication engagement with a plurality of classes (C1, C2) of implantable medical devices (10), the method comprising the steps of positioning a communication unit (11) of a programming device (12) within a communication range of an implantable medical device (10), and establishing a connection between the programming device (12) and the implantable medical device (10), wherein a class of the implantable medical device (10) is detected depending on a communication interface and/or a communication protocol of the implantable medical device (10), and wherein depending on the detected class of the implantable medical device (10) an auto-interrogation of the implantable medical device (10) is performedand/or a list of implantable medical devices (10) positioned within the communication range of the communication unit (11) of a programming device (12) is created.

## Description

The invention relates to a method for supporting a communication engagement with a plurality of classes of implantable medical devices. Furthermore, the invention relates to a programming device and system for supporting a communication engagement with a plurality of classes of implantable medical devices.

As implantable system architectures have evolved, so too have the communication modalities, protocols, and expectations for clinically-relevant user engagements central to their programming, interrogation, and supported active engagement monitoring capabilities. At a high level, this maturation has tended to facilitate an expansion of capabilities and/or data throughput to better support needs relevant to patient care.

With the arrival of leadless pacemaker systems in the cardiac rhythm management market, special needs have emerged, especially, in service of data relay across greater physical distances, i.e., between the implant and a wand-based programmer interface due to implant residence within the heart volume as opposed to shallower chest pocket installations common to transvenous implants.

The immediate transition to an ON-state communication behavior following the wake-up that stems from wand application is commonly referred to as an auto-interrogation dynamic.

Due to the shallow implantation depths of legacy devices, the data throughput associated with their communication engagements with the programmer can be relatively high-speed in nature and avoid a need to mandate that the programmer should serve as the initiator for any command/response interactions using frame-based architectures.

In contrast, for state-of-the-art leadless pacemaker systems, the communication infrastructure necessarily embodies slower data throughput modalities as a consequence of the implants' deeper residence within patient anatomy. This reduced cadence is best served by, in comparison to the norm for traditional pocket-based cardiac rhythm management products, leaning on the programmer to embody the command transmission role in a command/response relationship between the programmer and the leadless pacemaker.

There is also a general challenge regarding change-out procedures for leadless pacers that stems from difficulties and safety concerns associated with the explantation of chronically implanted devices nearing their end of service. This complication means that counter to legacy products, change-out interactions position at least two leadless implants within the range of the programmer wand routinely and thus the interactions with any single leadless implant need explicit management.

Furthermore, there exists a need for managing implant-specific communication interactions within a potential surrounding multitude of devices. Against a backdrop of prior product communication infrastructures having been developed in ways anchored in the assumptions that only a single pulse generator would reside within the range of a programmer wand at any given time and device explantation would prove feasible subject to change-out needs, supporting a clean strategy for meeting the needs of both past and emergent product needs presents a challenge.

Legacy cardiac rhythm management product communication is, most commonly, initiated by physically placing a programmer wand, i.e., a communication unit of a programming device over a single subcutaneously implanted pocket-based implant.

Legacy products only expect to represent the singularly relevant/visible implant in communication with the programmer. The surrounding communication infrastructure is thus commonly structured such that after initially awakening the lone in-range implant, typically by using a high-power wake-up signature from the wand, the implant immediately begins sending data content without any regard for possibly interfering with a data relay from possible alternative in-patient implants.

The distinct needs and behavioral responses in communication support for leadless and legacy cardiac rhythm management product products are not necessarily readily compatible through a shared interfacing design within clinicians' programmers without specific design considerations and management schemes.

It is therefore an object of the present invention to provide a method, a programming device and system for supporting a communication engagement with a plurality of classes of implantable medical devices.

The object is solved by a method for supporting a communication engagement with a plurality of classes of implantable medical devices having the features of claim 1. Furthermore, the object is solved by a programming device having the features of claim 12 as well as a system having the features of claim 15.

Further developments and advantageous embodiments are defined in the dependent claims.

The present invention provides a method for supporting a communication engagement with a plurality of classes of implantable medical devices. The method comprises positioning a communication unit of a programming device within a communication range of an implantable medical device.

Furthermore, the method comprises establishing a connection between the programming device and the implantable medical device, and automatically detecting a class of the implantable medical device depending on a communication interface and/or a communication protocol of the implantable medical device. Depending on the detected class of the implantable medical device an auto-interrogation of the implantable medical device is performed and/or a list comprising at least a list entry representing the implantable medical device positioned within the communication range of the communication unit of a programming device is created.

In addition, the present invention provides a programming device for supporting a communication engagement with a plurality of classes of implantable medical devices. The programming device comprises a communication unit, a processor, and a memory unit, wherein the memory unit comprises a computer-readable program which causes the processor to perform the following steps when executed on the processor: establishing a connection between the programming device and the implantable medical device when the communication unit (11) of the programming device (12) is positioned within a communication range of the implantable medical device, automatically detecting a class of the implantable medical device depending on a communication interface and/or a communication protocol of the implantable medical device, and depending on the detected class of the implantable medical device performing an auto-interrogation of the implantable medical device and/or creating a list comprising at least a list entry representing the implantable medical device.

In addition, the present invention provides a system for supporting a communication engagement with a plurality of classes of implantable medical devices comprising an implantable medical device and a programming device as described above.

An idea of the present invention is to provide a solution to the considerations noted above through the introduction of a novel discovery mode capability. This discovery mode combines the needs for selectively engaging with user-specified deeply implanted leadless pacemaker devices while also, in nominal cases, enabling the auto-interrogation needs of subcutaneously implanted pocket-based devices.

The invention thus provides a means for avoiding disruptions to the normal expectations relevant to communication support for a bulk of legacy products, i.e., a user simply places a wand (alternative term for communication unit of the programming device) over the implant and the programmer initiates a full interrogation process.

Furthermore, the invention provides a means for all multi-device engagements to be performed in a manner that can best serve helping a clinician direct system communication toward a single implant of interest. Moreover, the invention offers a means for effectively managing interactions where a mixture of modern and legacy implants may be presented and sidelines responses that would disrupt engagements already initiated with multi-device contextualized implants.

In an embodiment of the present invention, the plurality of classes of implantable medical devices includes a first class of implantable medical devices using a broad-band communication scheme or a Pulse-Position-Modulated (PPM) broad-band communication scheme, in particular auto-interrogation capable implantable medical devices, a second class of implantable medical devices using a narrow-band communication scheme or a Phase-Shift-Keying (PSK) narrow-band communication scheme, in particular leadless pacemakers, and/or a third class of implantable medical devices which cannot be automatically detected by the programming device.

In other words, in at least one embodiment of the present invention, the devices using broad-band communication are based on a pulse position scheme (PPM) and the devices using narrow-band communications are based on a phase shift keying (PSK) scheme. The programmer in this embodiment would selectively filter received signals from the broad-band devices to use information in the broader spectrum to analyze the characteristics that would identify this class of devices, while selectively filtering received signals from the narrow-band devices to allow correlating these signals with patterns that would identify this other class of devices.

In another embodiment of the present invention, if a first class of implantable medical devices using a broad-band communication scheme is detected prior to detecting any other device classes within the communication range of the communication unit of the programming device, auto interrogation of the detected implantable medical device is performed.

Thus, auto interrogation capable implantable medical devices can advantageously be auto interrogated within the common framework for supporting a communication engagement with a plurality of classes of implantable medical devices.

According to a further embodiment of the invention, the auto-interrogation of the implantable medical device comprises starting an application of the programming device, requesting a full configuration and statistics content from the implant, and starting the relay of medical data, in particular IEGM data. The framework for supporting a communication engagement with a plurality of classes of implantable medical devices according to the present invention thus fully supports auto interrogation capable implants.

In effect, this response dynamic realizes the same interactions as are presently in place for legacy CRM products. The auto interrogation amounts to the initiation of the appropriate device-relevant programmer application and an immediate sending of data from the implant to report its configuration, the contents of its on-board statistics, and an IEGM stream. All of this legacy behavior occurs subject to wand placement over a legacy device in cases where the wand had not already encountered a more modern implant.

According to a further embodiment of the invention, the detected at least one class of implantable medical devices may be output on an initial page of a graphical user interface of the programming device. The graphical user interface is thus able to present the detected class of implantable medical device within the plurality of potential classes of implantable medical devices.

According to a further embodiment of the invention, all classes of implantable medical devices may be accessible from the initial page of a graphical user interface of the programming device. The graphical user interface thus offers a means to communicate with a plurality of classes of implantable medical devices from a single starting page.

According to a further embodiment of the invention, in response to the detection of the auto-interrogation capable implantable medical device, the graphical user interface of the programming device transitions from the initial page to an open application page for the auto-interrogation capable implantable medical device.

As legacy implants are, for the most part, not expected to be implanted with other devices, this type of interaction avoids introducing added steps into the process of interrogation for most of the clinically-relevant device engagements one would expect with legacy devices, effectively keeping the same behaviors in place with which users are familiar.

According to a further embodiment of the invention, upon initiation of the application of the programming device, the configuration of the implantable medical device and its on-board statistics are made available along with a real-time feed of the IEGM data. The application of the program device thus provides a unified means to access a plurality of classes of implantable medical devices.

According to a further embodiment of the invention, if a second class of implantable medical devices using narrow-band communication is detected, the programming device creates the list comprising at least a list entry representing the implantable medical device detected within the communication range of the communication unit of the programming device.

When the wand (also known as communication unit of the programming device) first encounters a leadless pacer (or, more generally, a device enabled by a newer narrow-band, PSK-encoded communication scheme), a list of discovered devices is initiated. Even legacy devices are added to this list if they were seen by the wand after first encountering the more modern types. In effect, legacy (specifically PPM-enabled) devices that are "not seen first" by the wand, skip out on auto-interrogation and instead simply become user-selectable options within the wand-observed set of interrogatable implants. By adding these legacy (again, PPM-enabled) devices that are "seen after" the wand has already encountered PSK-enabled devices (rather than simply auto-interrogating any PPM-enabled device that the wand encounters), the system better assists user efforts to engage with PSK-enabled implants without having the system "highjack" attempts to communicate with the newer device class.

According to a further embodiment of the invention, once the list is initiated, one or more list entries representing respective further implantable medical devices of the first and/or second class detected within the communication range of the communication unit of the programming device are added to the list. Adding legacy implants to a list-based approach thus occurs in cases where a multi-implant aware device type has been encountered prior as the auto-interrogation dynamic described earlier prevails otherwise.

This ability to support the creation of a list where both legacy and modern devices serve as line items is made feasible by the use of narrow-band communication schemes for dialogue with the more modern devices as compared to the more broad-band communication schemes affiliated with legacy device communication links (approaches that are sufficiently distinguishable from the vantage of the interrogation/programming infrastructure that they can both be active and present without "jamming" the other). The legacy class of implants is oriented around the expectation that only a single legacy device interacts with the programmer at one time and thus is consistent with a frame-based communications protocol, while the newer class of devices that require support for multiple such devices in the same communications field take advantage of the command/response architecture to allow for an addressing scheme to provide targeted communications.

According to a further embodiment of the invention, the method further comprises selecting a list entry of the list for accessing a respective implantable medical device of the first and/or second class. This provides a simple to use tool for the user to manage a plurality of classes of implantable medical devices.

According to a further embodiment of the invention, upon selecting a list entry a subsequent startup of an implant-appropriate programmer application is driven requesting the full configuration and statistics content from the chosen implantable medical device, and starting the relay of IEGM data. Automatic startup of the implant appropriate programmer application conveniently automates the process of communicating with the implantable medical device.

According to a further embodiment of the invention, the method further comprises, if a desired class cannot be automatically detected, establishing the connection by searching for the desired class in a targeted manner by entering the desired class into the programming device (12).

It is thus possible that even older communication formats are in place within the system as built into long-lasting, still implanted products from more than 20 years ago are supported.

In such cases there may be an inability for the baseline wake-up and discovery phase of communication to readily recognize such implant types. There the graphical user interface preferably incorporates an added accessibility where the merged concepts from above can be deliberately sidelined by the clinician and a targeted search for the device class of interest can be enabled by means of a bail-out user selection. This capacity can be preferably enabled through button presses on the initial screen of the graphical user interface.

In an embodiment of the present invention, the graphical user interface is configured to display the list and to enable a selection of the list entry for accessing the respective implantable medical device.

In another embodiment of the present invention, the graphical user interface comprises a user prompt for searching for a desired class in a targeted manner by entering the desired class, if the desired class cannot be automatically detected.

The herein described features of the method for supporting a communication engagement with a plurality of classes of implantable medical devices are also disclosed for the programming device and the system for supporting a communication engagement with a plurality of classes of implantable medical devices and vice versa.

### DESCRIPTION OF THE DRAWINGS

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a flowchart of a method for supporting a communication engagement with a plurality of classes of implantable medical devices according to an embodiment of the invention; and
- Fig. 2: shows a diagram of a system for supporting a communication engagement with a plurality of classes of implantable medical devices according to an embodiment of the invention.

### DETAILED DESCRIPTION

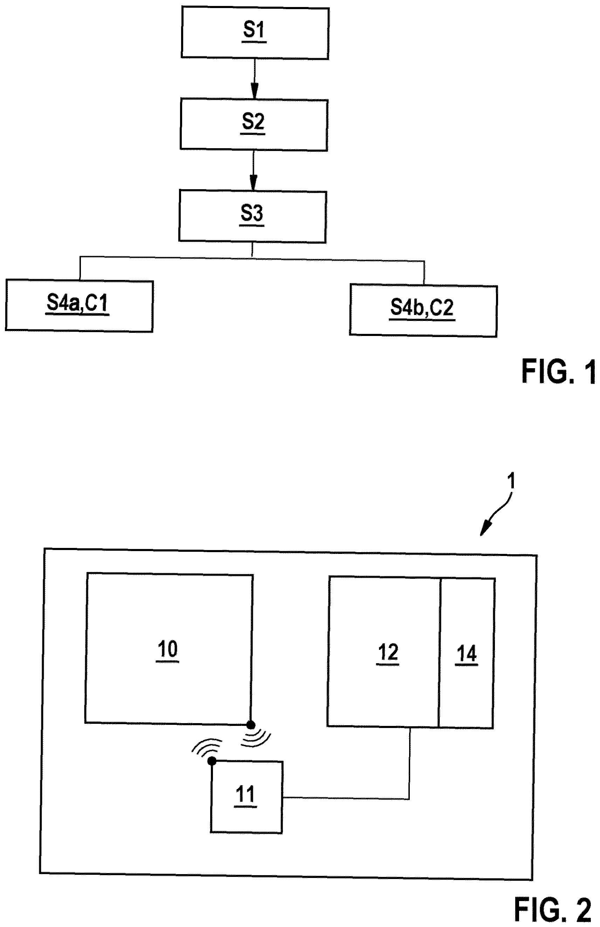

The method for supporting a communication engagement with a plurality of classes C 1, C2of implantable medical devices 10 shown in Fig. 1 comprises positioning S1 a communication unit 11 of a programming device 12 within a communication range of an implantable medical device 10.

The method furthermore comprises establishing S2 a connection between the programming device 12 and the implantable medical device 10, wherein a class C1, C2 of the implantable medical device 10 is detected depending on a communication interface and/or a communication protocol of the implantable medical device 10.

Depending on the detected class C1, C2 of the implantable medical device 10 an auto-interrogation of the implantable medical device 10 is performed S4a and/or a list of implantable medical devices 10 positioned within the communication range of the communication unit 11 of a programming device 12 is created S4b.

If a first class C1 of auto-interrogation capable implantable medical devices is detected, wherein if no other implantable medical device 10 or no other class is detected within the communication range of the communication unit 11 of the programming device 12, auto interrogation of the detected implantable medical device 10 is performed S4a. The auto-interrogation of the implantable medical device 10 further comprises starting an application of the programming device 12, requesting a full configuration and statistics content from the implant, and starting the relay of, in particular IEGM, medical data.

Moreover, the detected at least one class of implantable medical device 10 may be output on an initial page of a graphical user interface 14 of the programming device 12. All classes C 1, C2 of implantable medical devices 10 are accessible from the initial page of a graphical user interface 14 of the programming device 12, if at least one C2 device was encountered by the programming device prior to encountering any C 1 devices.

In response to the detection of the auto-interrogation capable implantable medical device 10, the graphical user interface 14 of the programming device 12 transitions from the initial page to an open application page for the auto-interrogation capable implantable medical device 10. Upon initiation of the application of the programming device 12, the configuration of the implantable medical device 10 and its on-board statistics are made available along with a real-time feed of the IEGM data.

If the programming device 12 detects a second class C2 of implantable medical devices, in particular a leadless pacemaker, first, the programming device 12 creates S4b the list of implantable medical devices 10 detected within the communication range of the communication unit 11 of the programming device 12. Once the list is initiated, implantable medical devices 10 of the first and/or second class detected within the communication range of the communication unit 11 of the programming device 12 are added to the list.

Upon creating the list of implantable medical devices 10 positioned within the communication range of the communication unit 11 of a programming device 12, any single entry of the list representing an implantable medical device 10 of the first and/or second class is selectable. A selection of any single entry of the list representing an implantable medical device 10 drives a subsequent startup of an implant-appropriate programmer application requesting the full configuration and statistics content from the chosen implantable medical device 10, and starting the relay of IEGM data.

If a desired class of implantable medical devices is not detected automatically, the connection to an implantable medical device of the desired class may be established by searching for the desired class in a targeted manner. For this case, the graphical user interface 14 of the programming device 12 provides a user prompt for establishing a connection with the implantable medical device 10 of the desired class.

Fig. 2 shows a schematic diagram of a system for supporting a communication engagement with a plurality of classes C1, C2 of implantable medical devices 10 according to an embodiment of the invention.

The system 1 comprises an implantable medical device 10, and a programming device 12 comprising a communication unit 11 configured to establish a connection between the programming device 12 and the implantable medical device 10, wherein a class of the implantable medical device 10 is detectable depending on a communication interface and/or a communication protocol of the implantable medical device 10.

Depending on the detected class of the implantable medical device 10 the programming device 12 is configured to perform an auto-interrogation of the implantable medical device 10 and/or to create a list of implantable medical devices 10 positioned within the communication range of the communication unit 11 of a programming device 12.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein. Therefore, it is the intent to cover all such modifications and alternate embodiments as may come within the true scope of this invention.

### Reference Signs

- 1: system
- 10: implantable medical device
- 11: communication unit
- 12: programming device
- 14: graphical user interface
- C1: first class
- C2: second class
- S1-S4b: method steps

## Claims

1. Method for supporting a communication engagement with a plurality of classes (C1, C2,) of implantable medical devices comprising the steps of:
positioning (S1) a communication unit (11) of a programming device (12) within a communication range of an implantable medical device (10); establishing (S2) a connection between the programming device (12) and the implantable medical device (10);
automatically detecting (S3) a class (C1, C2) of the implantable medical device (10) depending on a communication interface and/or a communication protocol of the implantable medical device (10); and
depending on the detected class (C1, C2) of the implantable medical device (10) performing (S4a) an auto-interrogation of the implantable medical device (10), and/or creating (S4b) a list comprising at least a list entry representing the implantable medical device (10) .

2. Method of claim 1, wherein the plurality of classes of implantable medical devices (10) includes a first class (C1) of implantable medical devices using a broad-band communication scheme or a Pulse-Position-Modulated (PPM) broad-band communication scheme, in particular auto-interrogation capable implantable medical devices, a second class (C2) of implantable medical devices using a narrow-band communication scheme or a Phase-Shift-Keying (PSK) narrow-band communication scheme, in particular leadless pacemakers, and/or a third class of implantbale medical devices which cannot be automatically detected by the programming device (12).

3. Method of claim 1 or 2, wherein if a first class (C1) of implantable medical devices using a broad-band communication scheme is detected prior to detecting any other device classes within the communication range of the communication unit (11) of the programming device (12), auto interrogation of the implantable medical device (10) is performed.

4. Method of any one of claims 1 to 3, wherein the auto-interrogation of the implantable medical device (10) comprises starting an application of the programming device (12), requesting a full configuration and statistics content from the implant, and starting the relay ofmedical data, in particular IEGM data.

5. Method of the preceding claim, wherein upon initiation of the application of the programming device (12), the configuration of the implantable medical device (10) and its on-board statistics are made available along with a real-time feed of the IEGM data.

6. Method of any one of the preceding claims, wherein if a second class (C2) of implantable medical devices using narrow-band communication is detected, the programming device (12) creates the list comprising at least a list entry representing the implantable medical device (10) detected within the communication range of the communication unit (11) of the programming device (12).

7. Method of claim 6, wherein once the list is initiated, one or more list entries representing respective further implantable medical devices (10) of the first and/or second class detected within the communication range of the communication unit (11) of the programming device (12) are added to the list.

8. Method of claim 6 or 7, further comprising: selecting a list entry of the list for accessing a respective implantable medical device (10) of the first and/or second class.

9. Method of any one of claims 6 to 8, wherein upon selecting a list entry a subsequent startup of an implant-appropriate programmer application is driven requesting the full configuration and statistics content from the chosen implantable medical device (10) and starting the relay of IEGM data.

10. Method of any one of the preceding claims, wherein, if a desired class cannot be automatically detected, establishing the connection by searching for the desired class in a targeted manner by entering the desired class into the programming device (12).

11. Method of any one of claims 8 to 10, wherein the step of selecting a list entry or the step of entering the desired class is performed by using a graphical user interface of the programming device (12).

12. Programming device (12) for supporting a communication engagement with a plurality of classes (C1, C2) of implantable medical devices (10) comprising:
a communication unit (11),
a processor, and
a memory unit, wherein the memory unit comprises a computer-readable programm which causes the processor to perform the following steps when executed on the processor:
establishing (S2) a connection between the programming device (12) and an implantable medical device (10) when the communication unit (11) of the programming device (12) is positioned within a communication range of the implantable medical device (10);
automatically detecting (S3) a class (C1, C2) of the implantable medical device (10) depending on a communication interface and/or a communication protocol of the implantable medical device (10); and
depending on the detected class (C1, C2) of the implantable medical device (10) performing (S4a) an auto-interrogation of the implantable medical device (10) and/or creating (S4b) a list comprising at least a list entry representing the implantable medical device (10).

13. Programming device (12) of claim 12, further comprising a graphical user interface configured to display the list and to enable a selection of the list entry for accessing the respective implantable medical device.

14. Programming device (12) of claim 13, wherein the graphical user interface comprises a user prompt for searching for a desired class in a targeted manner by entering the desired class, if the desired class cannot be automatically detected.

15. System (1) for supporting a communication engagement with a plurality of classes (C1, C2) of implantable medical devices (10) comprising:
an implantable medical device (10); and
a programming device (12) according to any of claims 12 to 14.
